Europäisches Patentamt

European Patent Office

Office européen des brevets

(11) Veröffentlichungsnummer: **0 272 507 B1**

# EUROPÄISCHE PATENTSCHRIFT

(45) Veröffentlichungstag der Patentschrift: **13.11.91**

(51) Int. Cl.⁵: **G01N 33/00, G01N 1/22**

(21) Anmeldenummer: **87117777.0**

(22) Anmeldetag: **02.12.87**

(54) **Verfahren und Anordnung zur Bestimmung mindestens einer Komponente eines Prüfgases.**

(30) Priorität: **20.12.86 DE 3643804**

(43) Veröffentlichungstag der Anmeldung:
**29.06.88 Patentblatt 88/26**

(45) Bekanntmachung des Hinweises auf die
Patenterteilung:
**13.11.91 Patentblatt 91/46**

(84) Benannte Vertragsstaaten:
**DE FR GB IT NL**

(56) Entgegenhaltungen:
**DE-A- 2 626 905**
**FR-A- 2 297 417**
**US-A- 4 117 713**
**US-A- 4 570 073**

**ELECTRONIQUE ET APPLICATIONS INDU-
STRIELLES, Band 242, Oktober 1977, Seiten
49-51, Paris, FR; U. ZELBSTEIN:
"L'odorographe du CNRS: Un appareil unique d'analyse chimique des substances
odorantes"**

(73) Patentinhaber: **Drägerwerk Aktiengesellschaft
Moislinger Allee 53-55
W-2400 Lübeck 1(DE)**

(72) Erfinder: **Leichnitz, Kurt, Dipl.-Ing.
Am Waldrand 42
W-2401 Gross Grönau(DE)**
Erfinder: **Matthiessen, Hans, Dipl.-Ing.
Heischbrook 22
W-2407 Gross Parin(DE)**

**Beschreibung**

Die Erfindung betrifft ein Verfahren zur Bestimmung einer Komponente sehr geringer Konzentration eines Prüfgases, welches in einer Förderleitung zur Anreicherung der Komponente über einen Probensammler geleitet wird, dessen angereicherte Probenmenge an einen Nachweissensor abgegeben wird. Außerdem wird eine Anordnung zur Durchführung des Verfahrens angegeben.

In der US-A-4 277 251 wird am Beispiel der Messung des Alkoholgehaltes in der Ausatemluft einer Person ein derartiges Verfahren angewendet, bei welchem die Atemgasprobe durch einen die Alkoholkomponente adsorbierenden Probensammler geleitet und in ihm gesammelt wird. Nach Beendigung der Probenahme wird der Probensammler beheizt, wodurch das adsorbierte Gas aus ihm ausgetrieben und einer angeschlossenen Meßeinheit zugeführt wird.

Bei der Verwendung der bekannten Methode erweist es sich jedoch als nachteilig, daß während des gesamten Vorganges für die Probensammlung keine Überwachung oder gar Messung des zu untersuchenden Gases auf seine Bestandteile hin durchgeführt werden kann. Der Meßvorgang wird somit auf zwei voneinander abhängige und zeitlich nacheinander folgende Arbeitsgänge verteilt, nämlich zunächst die Probensammlung, während welcher keine Messung durchgeführt werden kann, und die Probenuntersuchung, während welcher kein neuer Sammelvorgang stattfinden kann. Ein derartiges mit dem bekannten Meßverfahren arbeitendes Meßgerät ist also während des Probensammelns nicht meßbereit. Außerdem ist die bekannte Methode nur dann zuverlässig und aussagekräftig, wenn das zu untersuchende Prüfgas lediglich eine Komponente aufweist, die auch nachgewiesen werden soll. Denn dann sind das die Komponente speichernde Material des Probensammlers und die Empfindlichkeit des Meßgerätes aufeinander abstimmbar. Sind jedoch in dem Prüfgas mehrere Komponenten enthalten, welche in dem Probensammler angereichert werden können, so werden sie bei der nachfolgenden Austreibung ebenfalls gemeinsam dem Nachweissensor zugeführt. Ein getrennter, komponentenspezifischer Nachweis der Einzelkonzentrationen ist nicht möglich. Dies ist aber insbesondere dann wünschenswert, wenn Konzentrationen sehr geringer Größe von einzelnen Komponenten in dem Prüfgas vermutet werden. Diese können dann durch die sogenannte Querempfindlichkeit, d.h. der nichtgasartspezifischen Empfindlichkeit des Nachweissensors, bei einer gesammelten Analyse im Nachweissensor zu verfälschenden Meßergebnissen führen.

In der US-A-4,117,713 wird ein Verfahren und eine Geräteanordnung beschrieben, die dazu dient, Partikel aus dem Kühlgas eines Generators aufzuspüren und deren Zusammensetzung sowie Konzentration zu messen. Dazu wird das Kühlgas einem Nachweissensor zugeführt, der die Probe auf das Vorhandensein der betrachteten Partikelspezies überwacht. Liegt die Spezies vor, wird der Probengasstrom umgeleitet und die Partikel werden in einer Sammelkammer aufgefangen. Nach Abschluß der Sammelphase wird die Sammelphase mit partikelfreiem Gas durchspült und gleichzeitig durch eine Heizvorrichtung nach einem vorgebbaren Temperaturgang erwärmt. Die aus dem Sammelmedium der Sammelkammer ausgetriebenen Partikel werden durch den Nachweissensor erfaßt. Die Abhängigkeit der Meßsignalgröße von der Temperatur der Sammelkammer gibt Partikelart und -konzentration an. Nachteilig bei dieser bekannten Verfahrensanordnung ist die Tatsache, daß bei geringsten nachzuweisenden Partikelkonzentrationen der Nachweissensor erst gar nicht anspricht, um eine Sammelphase einzuleiten, so daß Messungen gar nicht erfolgen können. Außerdem sind zur Durchführung des Verfahrens mehrere Gasführungen nötig, die in einer aufwendigen Leitungsführung geschaltet werden müssen. Für die Austreibung der gesammelten Partikel ist ein gesondert aufbereitetes Trägergas notwendig.

Der vorliegenden Erfindung liegt somit die Aufgabe zugrunde, ein Verfahren der genannten Art so zu verbessern, daß schon während des Probensammelns ein Meßsignal zur Verfügung steht, und daß bei einem mehrkomponentigen Prüfgas sowohl die einzelnen Komponenten nachweisbar als auch eine komponentenspezifische Querempfindlichkeit des Nachweissensors kontrollierbar ist.

Die Lösung der Aufgabe wird durch die im kennzeichnenden Teil des Anspruchs 1 aufgeführten Merkmale erzielt.

Durch die Anwendung des Verfahrens wird es ermöglicht, ein mehrkomponentiges Prüfgas auf seine Bestandteile hin zu zerlegen und diese einzeln nachzuweisen, so daß Querempfindlichkeiten des Nachweissensors, wie sie beispielsweise bei elektrochemischen oder Halbleitersensoren besonders störend sind, ausgeschaltet und gasartspezifische Meßsignale erhalten werden. Dennoch kann gleichzeitig während des Probensammelns der Nachweissensor zum Zwecke der Warnung vor unerwünscht hohen Konzentrationen einzelner oder mehrerer Gaskomponenten eingesetzt werden.

In besonders einfacher Form kann der Probensammler aus einer Adsorptionsschicht bestehen, welcher während des Verfahrensschrittes der Probensammlung gekühlt und während des Verfahrensschrittes der Komponentenabgabe erwärmt wird. Komponenten, die gleichzeitig von der Adsorptionsschicht festgehalten werden, können durch Erwärmung des Probensammlers auf die jeweilige spezifische Desorptionstemperatur wieder ausgetrieben und einzeln nachgewiesen werden.

Eine Anordnung zur Anwendung des Verfahrens besitzt mindestens einen Probensammler, der dem Nachweissensor in der Förderleitung nachgeschaltet ist und mindestens eine Komponente des Prüfgases aussondert, wobei eine Fördereinrichtung in Reihe zu Probensammler und Nachweissensor vorgesehen ist. Dabei ist der Ausgang eines jeden Probensammlers über eine Abgangsleitung stromaufwärts vor den Nachweissensor an die Förderleitung angeschlossen. Somit kann während des Probensammelns der Nachweissensor zur Messung und Warnung herangezogen werden, und erst nach Abschluß des Probensammelns kann jeder einzelne Probensammler über seine Abgangsleitung mit dem Nachweissensor verbunden werden, so daß ein komponentenspezifisches Signal von dem Nachweissensor abgegeben wird. Dadurch kann das anfänglich gemessene Gesamtsignal auf Querempfindlichkeit des Nachweissensors hin untersucht und die Konzentration der einzelnen Komponenten bestimmt werden.

Zweckmäßigerweise sind die Abgangsleitungen in einem Wegeventil zusammengeführt, so daß sie von einer Steuereinheit je nach Bedarf an die Zuführungsleitung zugeschaltet oder von ihr abgetrennt werden können.

Eine besonders günstige Angestaltung für die Probensammler besteht darin, daß sie zu einer mehrstufigen Einheit zusammengefaßt sind, deren einzelne Stufen zur Selektion der jeweiligen Komponente in Reihe mit der Förderleitung angeordnet und zur Abgabe der selektierten Komponente parallel zueinander über eine eigene Abgangsleitung an die Förderleitung rückverbindbar sind.

Die Mehrstufigkeit kann dadurch verwirklicht sein, daß in einem Gehäuse schichtenförmig entsprechende Adsorptionsbetten in Reihe angeordnet, oder daß mehrere, komponentenspezifische Adsorptionsmaterialien untereinander vermischt sind, deren adsorbierte Gaskomponenten beispielsweise durch Erwärmung auf geeignete Temperaturen separat austreibbar und dem Nachweissensor zuführbar sind.

Ein Ausführungbeispiel der Erfindung wird anhand der schematischen Zeichnung dargestellt und im folgenden näher erläutert.

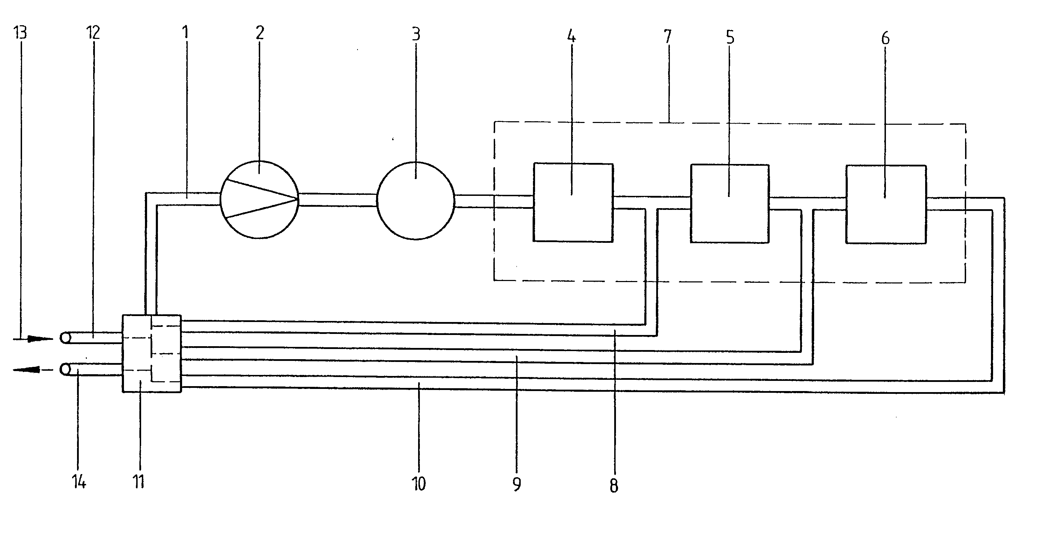

In der einzigen Figur ist innerhalb einer Förderleitung (1) eine Förderpumpe (2), ein Nachweissensor (3) sowie eine aus mehreren Stufen (4, 5, 6) gebildete Probensammlereinheit (7) hintereinander geschaltet angeordnet. Die Förderleitung (1) mündet zusammen mit den Abgagsleitungen (8, 9, 10) an den jeweiligen Ausgängen der Probensammlerstufen (4, 5, 6) in ein Wegeventil (11), welches zugleich mit einer Anschlußleitung (12) verbunden ist.

Die Steuerung der einzelnen Bauteile übernimmt eine nicht dargestellte Steuereinheit, deren Steuerablauf so geschaltet ist, daß während der Probensammlung die Förderpumpe (2) durch die Anschlußleitung (12) in Richtung des Ansaugpfeiles (13) das Prüfgas über den Sensor (3) und die nachfolgenden Probensammlerstufen (4, 5, 6) und die Abgangsleitung (10) zurück zum Wegeventil (11) und von dort in eine Auslaßleitung (14) leitet. Während des Sammelvorgangs werden einzelne Komponenten des Prüfgases in der jeweiligen Stufe (4, 5, 6) festgehalten, während der Sensor (3) ein Gesamtsignal aus dem Prüfgas mißt und anzeigt. Nach Beendigung der Probensammlung wird das Wegeventil (11) derart geschaltet, daß sowohl die Anschlußleitung (12) als auch die Auslaßleitung (14) von der Förderleitung (1) und den Abgangsleitungen (8, 9, 10) getrennt werden und nacheinander die Abgangsleitung (8, 9, 10) mit der Förderleitung (1) zu einer jeweiligen Ringleitung verbunden werden. Dabei wird die entsprechende Stufe (4, 5, 6) zur Abgabe der gesammelten Gaskomponente veranlaßt, was z. B. bei einer als Adsorptionsbett ausgebildeten Stufe durch Desorption mittels Wärmeeinwirkung erfolgen kann, wodurch der Sensor (3) eine während der Probensammlung ausgesonderte Komponente des Prüfgases messen und anzeigen kann.

**Patentansprüche**

1. Verfahren zur Bestimmung einer Komponente sehr geringer Konzentration eines Prüfgases, welches in einer Förderleitung (1) zur Anreicherung der Komponente über einen Probensammler (4, 5, 6) geleitet wird, dessen angereicherte Probenmenge an einen Nachweissensor (3) abgegeben wird, gekennzeichnet durch die Verfahrensschritte:

   a) das Prüfgas wird während des Probensammelns sowohl über den Nachweissensor (3) als auch über mindestens einen, zur Aussonderung mindestens einer Komponente vorgesehenen Probensammler (4, 5, 6) geleitet

   b) nach Unterbrechung der Prüfgasförderung gibt jeder der Probensammler (4, 5, 6) die von ihm ausgesonderte(n) Komponente(n) nacheinander an den Nachweissensor (3) ab.

2. Verfahren nach Anspruch 1, dadurch gekennzeichnet, daß der Probensammler (4, 5, 6) eine für die Aussonderung der Komponente geeignete Adsorptionsschicht enthält, welche während des Verfahrensschrittes a) gekühlt und während des Verfahrensschrittes b) erwärmt wird.

3. Anordnung zur Bestimmung einer Komponente sehr geringer Konzentration eines Prüfgases zur Durchführung des Verfahrens nach Anspruch 1, dadurch gekennzeichnet, daß dem Nachweissensor (3) in der Förderleitung (1) mindestens ein Probensammler (4, 5, 6) zur Aussonderung mindestens einer Komponente des Prüfgases nachgeschaltet, und in Reihe dazu eine Fördereinrichtung (2) vorgesehen, ist, und daß die Ausgänge eines jeden Probensammlers (4, 5, 6) über eine Abgangsleitung (8, 9, 10) stromaufwärts vor den Nachweissensor (3) an die Förderleitung (1) rückverbindbar sind.

4. Anordnung nach Anspruch 3, dadurch gekennzeichnet, daß die Abgangsleitungen (8, 9, 10) in einem Wegeventil (11) zusammengeführt sind.

5. Anordnung nach einem der Ansprüche 3 bis 4, dadurch gekennzeichnet, daß die Probensammler zu einer mehrstufigen Einheit (7) zusammengefaßt sind, deren einzelne Stufen (4, 5, 6) zur Selektion der jeweiligen Komponente in Reihe mit der Förderleitung (1) angeordnet zur Abgabe der selektiven Komponente parallel zueinander über eine eigene Abgangsleitung (8, 9, 10) an die Förderleitung (1) rückverbindbar sind.

**Claims**

1. Process for the determination of a component of very low concentration of a test gas which is conducted in a conveyor line (1) for the enrichment of the component via a sample collector (4, 5, 6), the enriched sample quantity of which is delivered to a detection sensor (3), characterized by the procedural steps:
a) during the sample collection the test gas is conducted both via the detection sensor (3) and via at least one sample collector (4, 5, 6) provided for the elimination of at least one component
b) after the interruption of the conveyance of test gas, each of the sample collectors (4, 5, 6) delivers the component(s) eliminated by it successively to the detection sensor (3).

2. Process according to claim 1, characterized in that the sample collector (4, 5, 6) contains an absorption layer suitable for the elimination of the component, the absorption layer being cooled during procedural step a) and heated during procedural step b).

3. Arrangement for the determination of a component of very low concentration of a test gas for carrying out the process according to claim 1, characterized in that at least one sample collector (4, 5, 6) is connected after the detection sensor (3) in the conveyor line (1) for the elimination of at least one component of the test gas, and in series therewith there is provided a conveyor device (2), and in that the outputs of each sample collector (4, 5, 6) can be return-connected to the conveyor line (1) by means of a departure line (8, 9, 10) upstream in front of the detection sensor (3).

4. Arrangement according to claim 3, characterized in that the departure lines (8, 9, 10) are brought together in a path valve (11).

5. Arrangement according to one of claims 3 to 4, characterized in that the sample collectors are combined into a multistage unit (7), the individual stages (4, 5, 6) of which for the selection of the respective component are arranged in series with the conveyor line (1) and can be return-connected to the conveyor line (1) for the delivery of the selective component in parallel with one another by means of their own departure line (8, 9, 10).

**Revendications**

1. Procédé pour déterminer un composant en très faible concentration dans un gaz à analyser qui est envoyé dans un conduit de transport (1) en vue d'enrichir le composant, par l'intermédiaire d'un collecteur d'échantillons (4, 5, 6), dont la quantité d'échantillons enrichis est transférée à un détecteur (3), caractérisé par les étapes de procédé suivantes :
a) le gaz à analyser est envoyé pendant la collecte des échantillons par le détecteur (3) ainsi que par au moins un collecteur d'échantillons (4, 5, 6) prévu pour séparer au moins un composant,
b) après interruption du transport de gaz à analyser, chacun des collecteurs d'échantillons (4, 5, 6) transfère successivement au détecteur (3) le (les) composant(s) qu'il a séparé(s).

2. Procédé selon la revendication 1, caractérisé en ce que le collecteur d'échantillons (4, 5, 6) contient une couche d'adsorption convenant à la séparation du composant, laquelle est refroidie pendant l'étape de procédé a) et chauffée pendant l'étape de procédé b).

3. Dispositif pour déterminer un Composant en

très faible concentration dans un gaz à analyser en vue de la mise en oeuvre du procédé selon la revendication 1, caractérisé en ce qu'il est monté en aval du détecteur (3), dans le conduit de transport (1), au moins un collecteur d'échantillons (4, 5, 6) pour séparer au moins un composant du gaz à analyser et en ce qu'il est prévu un dispositif de transport (2) en série avec ce collecteur et en ce que les sorties de chaque collecteur d'échantillons (4, 5, 6) peuvent être reliées en retour au conduit de transport (1), par un conduit de départ (8, 9, 10), en amont du détecteur (3).

4. Dispositif selon la revendication 3, caractérisé en ce que les conduits de départ (8, 9, 10) sont réunis dans une vanne de distribution (11).

5. Dispositif selon la revendication 3 ou 4, caractérisé en ce que les collecteurs d'échantillons sont réunis en une unité (7) à plusieurs étages dont les différents étages (4, 5, 6), montés en série avec le conduit de retour (1) pour la sélection du composant considéré, montés en parallèle entre eux pour le transfert du composant sélectif, peuvent être reliés en retour au conduit de transport (1), par leur propre conduit de départ (8, 9, 10).